# EUROPÄISCHE PATENTSCHRIFT

(11) **EP 3 181 548 B1**
(45) Veröffentlichungstag und Bekanntmachung des Hinweises auf die Patenterteilung: **21.10.2020**
(21) Anmeldenummer: 17154943.9
(22) Anmeldetag: 22.01.2005
(51) Int. Cl.: C07C 263/10, C07C 263/20, C07C 265/14, C08G 18/76

(54) **HERSTELLUNG VON DI- UND POLYISOCYANATEN DER DIPHENYLMETHAN-REIHE MIT DEFINIERTEM GEHALT AN HYDROLYSIERBAREM CHLOR**
PRODUCTION OF DI- AND POLYISOCYANATES OF THE DIPHENYLMETHANE SERIES HAVING A DEFINED CONTENT OF HYDROLYSABLE CHLORINE
FABRICATION DE DI- ET POLYISOCYANATES DE LA SÉRIE DES DIPHÉNYLMÉTHANES AYANT UNE TENEUR DÉFINIE EN CHLORE HYDROLYSABLE

(30) Priorität: 04.02.2004 DE 102004005320
(43) Veröffentlichungstag der Anmeldung: 21.06.2017
(62) Teilanmeldung aus: 05001280.6
(73) Patentinhaber: Covestro Deutschland AG, 51373 Leverkusen (DE)
(72) Erfinder: Liman, Ulrich, 40764 Langenfeld (DE); Müller, Heinz-Herbert, 47809 Krefeld (DE); Vieler, Robert, 41542 Dormagen (DE); Echterhoff, Ralf, 41539 Dormagen (DE)
(74) Vertreter: Levpat

(56) Entgegenhaltungen:
- WO-A-99/54289
- WO-A-02/070581
- DE-A1- 1 938 384
- DE-A1- 3 145 010
- DE-B- 1 138 040

## Beschreibung

Die vorliegende Erfindung betrifft ein Verfahren zur Herstellung von Di- und Polyisocyanaten der Diphenylmethanreihe und deren Mischungen.

Die Erfindung umfasst ein Verfahren zur Herstellung von Di- und Polyisocyanaten der Diphenylmethanreihe und Gemischen daraus, bei dem man
a) Anilin und Formaldehyd in Gegenwart eines sauren Katalysators zu Di- und Polyaminen der Diphenylmethanreihe umsetzt, und
b) die Di- und Polyamine der Diphenylmethanreihe gegebenenfalls in Gegenwart eines Lösungsmittels phosgeniert, wobei man ein rohes Di- und Polyisocyanat erhält, und
c) aus dem rohen Di- und Polyisocyanat destillativ wenigstens eine Fraktion enthaltend mindestens 5 Gew.-% 3-Kern- und höherkerniges MDI sowie wenigstens eine Fraktion enthaltend mindestens 95 Gew.-% 2-Kern-MDI abtrennt, und
d) den Wert für hydrolysierbares Chlor der Fraktion enthaltend mindestens 95 Gew.-% 2-Kern-MDI analytisch bestimmt, und
e) die Fraktion enthaltend mindestens 95 Gew.-% 2-Kern-MDI mit einer sauren Verbindung, die organische Säurechloride und/oder N-Chloracetamid umfasst, versetzt, so dass der gewünschte Wert für hydrolysierbares Chlor gezielt eingestellt wird.

Aromatische Isocyanate sind sehr wichtige Rohstoffe für die Herstellung von Polyurethan-Werkstoffen. Hierbei spielen die Di- und Polyisocyanate der Diphenylmethanreihe (MDI) die mengenmäßig größte Rolle.

Unter Polyisocyanaten der Diphenylmethanreihe werden Isocyanate und Isocyanatgemische folgenden Typs verstanden: 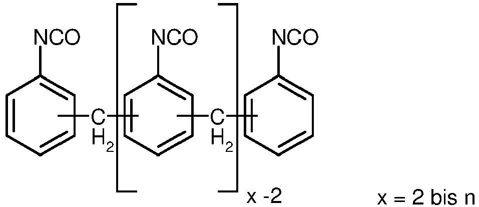 wobei n für eine natürliche Zahl > 0 steht.

Analog werden unter Polyaminen der Diphenylmethanreihe Verbindungen und Verbindungsgemische folgenden Typs verstanden:

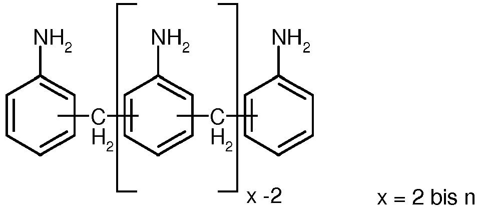

Es ist bekannt, dass Di- und Polyisocyanate der Diphenylmethanreihe (MDI) durch Phosgenierung der entsprechenden Di- und Polyamine der Diphenylmethanreihe (MDA) hergestellt werden. Die Di- und Polyamine der Diphenylmethanreihe selbst werden durch Kondensation von Anilin und Formaldehyd hergestellt. Durch Phosgenierung der Diamine der Diphenylmethanreihe erhält man die entsprechenden Diisocyanate 2,2'-MDI, 2,4'-MDI und 4,4'-MDI, die in der Fachwelt als 2-Kern-MDI (Diisocyanate der Diphenylmethanreihe) beschrieben werden. Während der Kondensation von Anilin und Formaldehyd reagiert das 2-Kern-MDA (Methylendiphenyldiamin MDA) jedoch auch noch weiter mit Formaldehyd und Anilin zu höherkernigen MDA-Typen, die nach der Phosgenierung den Mehrkerngehalt im polymeren MDI (Polyisocyanate der Diphenylmethanreihe) darstellen.

Für viele praktische Produktanwendungen ist es nun bevorzugt, einen definierten Säuregehalt im MDI einzustellen. Diese Standardisierung nivelliert Reaktivitätsschwankungen, die durch Nebenverbindungen in der Reaktionskette vom Anilin zum MDI entstehen. Diese Nebenverbindungen haben einen Einfluss auf den Säuregehalt des MDI bei der Verarbeitung mit H-aktiven Verbindungen wie die in der Polyurethan-Chemie eingesetzten Polyole.

In der Praxis wird der Säuregehalt im MDI als Acidität im polymeren MDI (PMDI) und/oder monomeren MDI (MMDI) oder als hydrolysierbares Chlor im MMDI ausgedrückt. Dieser Aciditäts-Wert wird in der Praxis durch Reaktion von MDI mit niederen Alkoholen, z.B. Methanol, ermittelt (siehe z.B. ASTM D5523-94 für monomeres MDI oder ASTM5629-99 bzw. 6099-03 für polymeres MDI).

Der Säuregehalt im MDI wird nach dem derzeitigen Stand der Technik durch die Prozessverfahrensparameter bei der Herstellung der MDA-Basen durch die Bildung von Nebenkomponenten im MDA, die von den jeweiligen MDA-Prozessparametern abhängt, und bei der Phosgenierung und der anschließenden Aufarbeitung bestimmt. Aufgrund der mehrstufigen Reaktionskette und der Schwankungen und Änderungen des Durchsatzes sowie der Prozessparameter während der Herstellung führt dies in der Praxis zu unvermeidbaren Schwankungen im Säuregehalt. Dabei werden üblicherweise Werte für den Säuregehalt im MDI als Acidität von MDI von bis zu 500 ppm Säure (berechnet als HCl) erreicht. Dabei ergeben sich jedoch mitunter Abweichungen von der gewünschten Acidität. Diese Schwankungen in der Acidität innerhalb einer hergestellten MDI-Produktqualität führen zu unerwünschten Abweichungen im Reaktionsverhalten des MDI bei der späteren Herstellung der Polyurethane.

DE-A-1 138 040 befasst sich mit einem Verfahren zur Entfernung von hydroylsierbarem Chlor und Verringerung der Acidität aus bzw. in organischen Isocyanaten (vgl. Titel, Patentanspruch 1).

DE 31 45 010 A1 befasst sich mit einem Verfahren zur Herstellung von hochreinem 4,4'-MDI durch Einhaltung bestimmter Destillationsbedingungen (siehe Zusammenfassung, Patentanspruch 1).WO 02/070581 A1 befasst sich mit einem Verfahren zur Bereitstellung von 2,4'-MDI ohne die Qualität des Gesamtverfahrens und die Zusammensetzung der übrigen MDI-Produkte nachteilig zu beeinflussen (S. 3, Z. 32 bis 40). Die Standardisierung von MDI-Partien bzgl. ihres Säuregehalts ist nicht Gegenstand des in WO 02/070581 A1 beschriebenen Verfahrens.

DE 1 938 384 befasst sich mit einem Verfahren zur Verringerung des Anteils an sauren, Chlor enthaltenden Verbindungen in MDI durch Schmelzkristallisation (vgl. Titel, Patentanspruch 1).

WO 99/54289 A1 befasst sich mit einem Verfahren zur Herstellung von Mischungen aus Diphenylmethandiisocyanaten und Polyphenylen-polymethylen-polyisocyanaten mit vermindertem Gehalt an chlorierten Nebenprodukten und verminderter Jodfarbzahl durch zweistufige Umsetzung der entsprechenden Mischungen aus Diphenylmethan-diaminen und Polyphenylen-polymethylenpolyaminen mit Phosgen in Gegenwart mindestens eines inerten organischen Lösungsmittels bei erhöhter Temperatur, nach beendeter Phosgenierung Abtrennung des überschüssigen Phosgens und Lösungsmittels und thermischer Behandlung des Reaktionsproduktes, dadurch gekennzeichnet, dass die Massenverhältnisse von Phosgen zu Chlorwasserstoff im Verweilzeitapparat der zweiten Stufe der Phosgenierung gleichzeitig in der Flüssigphase 10-30:1 und in der Gasphase 1-10:1 betragen.

Generell gilt, dass sich bei steigendem Säuregehalt die Reaktivität des MDI zum Polyol verlangsamt. Da jedoch bei der Polyurethan-Verarbeitung die Reaktivität in der Regel durch die Katalyse im Polyol definiert werden soll, ist es essentiell für eine prozesssichere und reproduzierbare Verarbeitung, dass die MDI Komponente eine konstante Reaktivität hat. Der bei der Herstellung nach dem Stand der Technik erreichte Säuregehalt (insbesondere beim MMDI) kann sogar so niedrig sein, dass ungewünschte Nebenreaktionen bei der Polyurethan-Verarbeitung auftreten (z.B. alkalisch katalysierte Polyisocyanaurat Reaktion).

Im Markt werden daher die verschiedenen gehandelten MDI-Produkte mit einem Spezifikationswert für die Acidität bzw. dem Gehalt an hydrolisierbarem Chlor (HC-Wert) spezifiziert. Dabei werden üblicherweise die Analysen der Acidität gemäß den oben genannten Testmethoden ASTM durchgeführt, die den Gehalt an freigesetzter Säure bei Umsetzung mit H-aktiven Verbindungen beschreiben. In der Regel wird dieser Wert als HCl berechnet.

Die Aufgabe der vorliegenden Erfindung ist daher, ein einfaches Verfahren zur Herstellung von Di- und Polyisocyanaten der Diphenylmethanreihe und Gemischen daraus mit definiertem und konstantem Wert für den Säuregehalt (Gehalt an hydrolysierbarem Chlor) bereit zustellen, mit dem eine konstante MDI-Produktqualität zuverlässig gewährleistet werden kann.

Die Erfindung betrifft ein Verfahren zur Herstellung von Di- und Polyisocyanaten der Diphenylmethanreihe und Gemischen daraus, bei dem man
a) Anilin und Formaldehyd in Gegenwart eines sauren Katalysators zu Di- und Polyaminen der Diphenylmethanreihe umsetzt, und
b) die Di- und Polyamine der Diphenylmethanreihe gegebenenfalls in Gegenwart eines Lösungsmittels phosgeniert, wobei man ein rohes Di- und Polyisocyanat erhält, und
c) aus dem rohen Di- und Polyisocyanat destillativ wenigstens eine Fraktion enthaltend mindestens 5 Gew.-% 3-Kern- und höherkerniges MDI sowie wenigstens eine Fraktion enthaltend mindestens 95 Gew.-% 2-Kern-MDI abtrennt, und
d) den Wert für hydrolysierbares Chlor der Fraktion enthaltend mindestens 95 Gew.-% 2-Kern-MDI analytisch bestimmt, und
e) die Fraktion enthaltend mindestens 95 Gew.-% 2-Kern-MDI mit einer sauren Verbindung, die organische Säurechloride und/oder N-Chloracetamid umfasst, versetzt, so dass der gewünschte Wert für hydrolysierbares Chlor gezielt eingestellt wird.

Anschließend werden die in Schritt e) erzeugten Fraktionen bevorzugt in Behälter abgefüllt und stehen dann als Isocyanatkomponente mit gezielt eingestelltem Wert für hydrolysierbares Chlor für die Herstellung von modifizierten Isocyanaten, Abmischprodukten mit anderen Isocyanatfraktionen, Prepolymeren und Polyurethanen zur Verfügung.

Durch die Zugabe der sauren organischen oder anorganischen Verbindungen als Additiv zum MDI in Schritt e) wird erreicht, dass die spezifizierten Werte des MDI unabhängig vom Herstellprozess exakt und reproduzierbar eingestellt werden können.

Die Erfindung betrifft somit ein Verfahren zur Herstellung von MDI und deren Gemischen, bei dem der Wert für hydrolysierbares Chlor des MDI bzw. deren Gemische durch Zugabe saurer, insbesondere halogenhaltiger und vor allem chlorhaltiger Verbindungen nach der destillativen Auftrennung des rohen MDI-Gemisches in die Isomeren bzw. Isomerengemische gezielt eingestellt wird. Dabei wird durch die gezielte Einstellung des Werts für hydrolysierbares Chlor eine konstante Reaktivität des MDI gewährleistet, ohne die bisher vorhandenen Produktspezifikationen zu verletzen.

Das im erfindungsgemäßen Verfahren eingesetzte Polyamin bzw. Polyamingemisch der Diphenylmethanreihe wird in Schritt a) durch Kondensation von Anilin und Formaldehyd in Gegenwart eines sauren Katalysators erhalten (H. J. Twitchett, Chem. Soc. Rev. 3(2), 209 (1974); W. M. Moore in: Kirk-Othmer Encycl. Chem. Technol., 3rd. Ed., New York, 2, 338-348 (1978)). Für das erfindungsgemäße Verfahren ist es dabei unerheblich, ob Anilin und Formaldehyd zunächst in Abwesenheit des sauren Katalysators vermischt werden und der saure Katalysator anschließend zugesetzt wird oder ob ein Gemisch aus Anilin und saurem Katalysator mit Formaldehyd zu Reaktion gebracht wird.

Geeignete Polyamingemische der Diphenylmethanreihe werden üblicherweise erhalten durch Kondensation von Anilin und Formaldehyd im Stoffmengenverhältnis 20 bis 1,6, bevorzugt 10 bis 1,8 sowie einem Stoffmengenverhältnis von Anilin und saurem Katalysator von 20 bis 1, bevorzugt 10 bis 2.

Formaldehyd wird technisch üblicherweise als wässrige Lösung eingesetzt. Hierbei kann der Wassergehalt von 1 bis 95% variieren. Bevorzugt wird eine wässrige Lösung mit 50 bis 80% Wasser eingesetzt. Es können jedoch auch andere methylengruppenliefernde Verbindungen wie z.B. Polyoxymethylenglykol, *para*-Formaldehyd oder Trioxan eingesetzt werden.

Als saure Katalysatoren haben sich starke organische und vorzugsweise anorganische Säuren bewährt. Geeignete Säuren sind beispielsweise Salzsäure, Schwefelsäure, Phosphorsäure, und Methansulfonsäure. Bevorzugt wird Salzsäure eingesetzt.

In einer bevorzugten Ausführungsform des Verfahrens wird Anilin und saurer Katalysator zunächst vermischt. Dieses Gemisch wird, ggf. nach Abfuhr von Wärme, in einem weiteren Schritt mit Formaldehyd bei Temperaturen zwischen 20°C und 100°C, bevorzugt bei 30°C bis 70°C in geeigneter Weise vermischt und anschließend in einem geeigneten Verweilzeitapparat einer Vorreaktion zu unterzogen. Die Vorreaktion erfolgt bei Temperaturen zwischen 20°C bis 100°C, vorzugsweise im Temperaturbereich 30°C bis 80°C. Im Anschluss an Vermischung und Vorreaktion wird die Temperatur des Reaktionsgemisches in Stufen oder kontinuierlich und ggf. unter Überdruck auf eine Temperatur von 100°C bis 250°C, bevorzugt auf 100°C bis 180°C, besonders bevorzugt auf eine Temperatur von 100°C bis 160°C gebracht.

Es ist jedoch ebenfalls möglich, in einer anderen Ausführungsform des Verfahrens Anilin und Formaldehyd zunächst in Abwesenheit des sauren Katalysators im Temperaturbereich von 5°C bis 130°C, bevorzugt von 40°C bis 100°C, besonders bevorzugt von 60°C bis 90°C, zu vermischen und zur Reaktion zu bringen. Hierbei bilden sich Kondensationsprodukte aus Anilin und Formaldehyd (sog. Aminal). Im Anschluss an die Aminalbildung kann im Reaktionsgemisch vorhandenes Wasser durch Phasentrennung oder andere geeignete Verfahrensschritte, beispielsweise durch Destillation, entfernt werden. Das Kondensationsprodukt wird dann in einem weiteren Verfahrensschritt mit dem sauren Katalysator in geeigneter Weise vermischt und in einem Verweilzeitapparat bei 20°C bis 100°C, bevorzugt 30°C bis 80°C einer Vorreaktion unterzogen. Anschließend wird die Temperatur des Reaktionsgemisches in Stufen oder kontinuierlich und ggf. unter Überdruck auf eine Temperatur von 100°C bis 250°C, bevorzugt auf 100°C bis 180°C, besonders bevorzugt auf eine Temperatur von 100°C bis 160°C gebracht.

Zur Aufarbeitung des sauren Reaktionsgemisches wird das Reaktionsgemisch nach dem Stand der Technik mit einer Base neutralisiert. Nach dem Stand der Technik erfolgt die Neutralisation üblicherweise bei Temperaturen von beispielsweise 90 - 100°C (H.J. Twitchett, Chem. Soc. Rev. 3(2), 223 (1974)). Als Basen sind beispielsweise geeignet die Hydroxide der Alkali- und Erdalkalielemente. Vorzugsweise kommt wässrige NaOH zur Anwendung.

Im Anschluss an die Neutralisation wird nach dem Stand der Technik die organische von der wässrigen Phase durch geeignete Verfahren (z.B. Phasentrennung in einer Scheideflasche) getrennt. Diese Trennung von organischer und wässriger Phase kann bei der selben Temperatur erfolgen, bei der die Neutralisation des sauren Umlagerungsgemisches erfolgte. Die nach Abtrennung der wässrigen Phase verbleibende produktenthaltende organische Phase wird nach dem Stand der Technik zur Abtrennung von Salzen und überschüssiger Base einer Wäsche unterzogen. Anschließend wird die gereinigte organische Phase von überschüssigem Anilin und anderen im Gemisch vorhandenen Stoffen (z.B. weiteren Lösungsmitteln) durch geeignete physikalische Trennverfahren wie z.B. Destillation, Extraktion oder Kristallisation befreit.

Das so erhaltene Polyamin der Diphenylmethanreihe (Roh-MDA) aus Schritt a) wird im Schritt b) nach den bekannten Methoden mit Phosgen in einem inerten organischen Lösungsmittel zu den entsprechenden Isocyanaten umgesetzt. Das Molverhältnis von Roh-MDA zu Phosgen wird zweckmäßigerweise so bemessen, dass pro Mol NH₂-Gruppe 1 bis 10 Mol, vorzugsweise 1,3 bis 4 Mol Phosgen in der Reaktionsmischung vorliegen. Als inerte Lösungsmittel haben sich chlorierte, aromatische Kohlenwasserstoffe wie z.B. Monochlorbenzol, Dichlorbenzole, Trichlorbenzole, die entsprechenden Toluole und Xylole sowie Chlorethylbenzol bewährt. Insbesondere Anwendung finden als inerte organische Lösungsmittel Monochlorbenzol, Dichlorbenzol oder Mischungen dieser Chlorbenzole. Die Menge an Lösungsmittel wird vorzugsweise so bemessen, dass die Reaktionsmischung einen Isocyanatgehalt von 2 bis 40 Gew.-%, vorzugsweise zwischen 5 uns 20 Gew.-%, bezogen auf das Gesamtgewicht der Reaktionsmischung, aufweist. Die Reaktion von Roh-MDA mit Phosgen wird hierbei bei Temperaturen von 50 bis 250°C und bei Drücken von Umgebungsdruck bis hin zu 50 bar durchgeführt. Bevorzugt wird bei 70 bis 180°C und 2 bis 20 bar gearbeitet.

Nach beendeter Phosgenierung werden aus der Reaktionsmischung das überschüssige Phosgen, das inerte organische Lösungsmittel, die gebildete HCl oder Mischungen davon durch geeignete Verfahren (z.B. Destillation) abgetrennt. Hierzu wird der Druck schrittweise bis ins Vakuum reduziert und der verbliebene Phosgenüberschuss und die gebildete HCl verdampft und abgetrennt. Anschließend wird bevorzugt per Verdampfung das Lösungsmittel schrittweise reduziert unter weiterer Reduzierung des absoluten Drucks auf bis zu 1 mbar, bevorzugt auf bis zu 5 mbar. Parallel dazu wird die Temperatur erhöht bis das Lösungsmittel fast vollständig bis auf weit weniger als 0,1% entfernt ist. Letztlich enthält man in diesem Schritt b) ein rohes Di- und Polyisocyanat (rohes MDI-Gemisch).

Die Auftrennung kann destillativ unter Vakuum oder durch Kristallisation erfolgen. Hierbei ist auch eine Kombination beider Prozesse möglich.

In Schritt c) wird aus dem rohen Di- und Polyisocyanat destillativ wenigstens eine Fraktion enthaltend mindestens 5 Gew.-% 3-Kern- und höherkerniges MDI sowie wenigstens eine Fraktion enthaltend mindestens 95 Gew.-% 2-Kern-MDI abgetrennt.

In Schritt d) wird der Wert für hydrolysierbares Chlor der in Schritt c) hergestellten Fraktion enthaltend mindestens 95 Gew.-% 2-Kern-MDI analytisch bestimmt. Für monomeres MDI erfolgt die Bestimmung der Acidität als Bestimmung für den Wert für hydrolysierbares Chlor. Diese Bestimmung kann nach der in Beispiel 1 angegebenen Methode erfolgen.

In PMDI können bei einer mittleren Acidität von z.B. ca. 100 ppm üblicherweise Aciditätsschwankungen im Bereich von bis zu 50% auftreten, die durch Schwankungen oder Änderungen des Durchsatzes und der Prozessparameter der Herstellung bzw. in unterschiedlichen Produktionsanlagen auftreten. Wünschenswert ist eine Aciditätsschwankung unter 5%. Dies würde dadurch erreicht, dass dem MDI je nach gegebener Acidität das aciditätsaktive Zusatzmittel in einer Menge zugesetzt wird, so dass die Acidität oberhalb des Maximalwertes der prozessbedingten Schwankungsbreite des jeweiligen MDI liegt. In diesem Beispiel läge der Maximalwert somit bei 100 ppm + 50 ppm = 150 ppm. Die technisch verfügbaren Einrichtungen zur Dosierung der sauren Verbindung sind so exakt, dass die dann erreichte Schwankungsbreite vernachlässigbar gering ist. Analog stellt sich der Fall beim erfindungsgemäß bezüglich seines Werts für hydrolysierbares Chlor einzustellenden MMDI dar mit einer ungefähren Acidität (ausgedrückt als Wert für hydrolysierbares Chlor) von z.B. 10 ppm mit Schwankungen von bis zu 100 %. Hier wird in analoger Weise die saure Verbindung als aciditätsaktives Additiv zum MMDI zugesetzt.

In Schritt e) wird die in Schritt c) erhaltene Fraktion enthaltend mindestens 95 Gew.-% 2-Kern-MDI mit einer sauren Verbindung versetzt, so dass der gewünschte Wert für hydrolysierbares Chlor erreicht wird.

Hierzu werden flüssige oder feste organische Verbindungen verwendet, die den Wert für hydrolysierbares Chlor erhöhen, wie z.B. Benzotrichlorid, Benzoylchlorid, Phthalsäuredichlorid, Terephthalsäuredichlorid, Isophthalsäuredichloride, p-Toluolsulfonsäurechlorid, oder andere organische Säurechloride. Bevorzugt werden dabei organische Verbindungen eingesetzt, die sich gut in MDI lösen und zugleich einen niedrigen Dampfdruck aufweisen und die sonstigen Eigenschaften der gewünschten Isocyanat-Additionsprodukte im Fertigteil nicht ändern. Geeignete organische saure Verbindungen, die den Wert für hydrolysierbares Chlor erhöhen sind Essigsäurechlorid, Trichloressigsäurechlorid oder N-Chloracetamid, Benzoylchlorid, Phthalsäurechlorid, Terephthalsäuredichlorid, Isophthtalsäuredichlorid, aromatische, aliphatische oder cycloaliphatische Carbaminsäurechloride wie beispielsweise N-Phenylcarbamylchlorid, tert.-Butylcarbamylchlorid, Tosylchlorid oder Gemische daraus.

Bevorzugt werden Benzoylchlorid, Phthalsäuredichlorid, Terephthalsäuredichlorid, Butylcarbamoylchloride eingesetzt. Vorzugsweise werden bei der Einstellung des Werts für hydrolysierbares Chlor für MDI und deren Derivaten und Modifizierungen, wie Allophanaten, Carbodiimiden, Uretoniminen, Uretdionen und prepolymeren Urethanen und/oder prepolymeren Harnstoffen aromatische bzw. aliphatische Säurechloride oder Carbamylchloride eingesetzt. Bevorzugt werden dabei aromatische, bzw. aliphatischen Säurechloride oder Carbamylchloride eingesetzt, die bei der Herstellung der Polyurethane nicht stören und einen niedrigen Dampfdruck aufweisen. Bevorzugt eingesetzt werden Benzoylchlorid, Terephthalsäuredichlorid, Isophthalsäuredichlorid, Phenylcarbamylchlorid, tert. Butylcarbamylchlorid.

Die Einbringung der sauren Verbindung kann direkt in das gewünschte MDI erfolgen oder in Form einer höher konzentrierten Stammlösung, die bevorzugt mit dem entsprechenden MDI angesetzt wird, das die Spezifikation des gewünschten MDI's nicht ändert.

Durch das erfindungsgemäße Verfahren wird erreicht, dass das erhaltene MDI einen definierten Gehalt an hydrolysierbarem Chlor aufweist, und daher ein konstantes und definiertes Reaktionsverhalten gegenüber H-aktiven Verbindungen ergibt. Dies wirkt sich positiv auf eine störungsfreie Verarbeitung bei der Serienfertigung von daraus hergestellten Polyadditions Fertigteilen aus.

Die nach dem erfindungsgemäßen Verfahren hergestellten MDI-Produkte mit definiertem Wert für hydrolysierbares Chlor können mit einem oder mehreren Gemischen enthaltend aromatische Isocyanate abgemischt werden. Für die Abmischung können neben den bereits erwähnten, bevorzugt eingesetzten Gemischen enthaltend Di- und/oder Polyisocyanate der Diphenylmethanreihe bevorzugt auch Gemische enthaltend Toluylendiisocyanat (zum Beispiel 2,4-TDI, 2,6-TDI) oder Gemische enthaltend Naphthalindiisocyanat (zum Beispiel 1,5-NDI) oder Gemische aus diesen Isocyanaten eingesetzt werden. Grundsätzlich können für die Abmischung aber auch andere aromatische und/oder aliphatische Mono-, Di-, Tri- oder höherfunktionellen Isocyanate eingesetzt werden.

Die nach dem erfindungsgemäßen Verfahren hergestellten MDI Produkte mit definiertem Werten für hydrolysierbares Chlor können zu den üblichen Modifizierungsreaktionen von Isocyanaten benutzt werden, wie z.B. zur Carbodiimidisierung durch Temperaturerhöhung oder Zugabe von Phospholenoxiden und zur auf den Carbodiimiden aufbauenden Bildung von Uretoniminen, oder zur Herstellung von Prepolymeren aus OH-funktionellen Polyestern, C₂/C₃/C₄-Polyethern, Uretdionen, Allophanaten, Biureten oder Harnstoffderivaten.

Die nach dem erfindungsgemäßen Verfahren hergestellten Fraktionen von Diisocyanaten der Diphenylmethenreihe finden Verwendung unter anderem als Abmischkomponenten für andere Di- und Polyisocyanate der MDI-Reihe und/oder anderen Isocyanaten zur Herstellung von Isocyanatkomponenten für den Einsatz in der Herstellung von Polyurethanenen. Die aus diesen Abmischprodukten herzustellenden Fertigteile decken das gesamte Feld der Polyurethan-Chemie ab. Beispielhaft genannt sind hier:
- Hartschaumstoffe für die Isolier und Kühlgeräteindustrie
- Verpackungsschaum
- Weichschaum und Weichformschaum für Möbel und Autoindustrie
- Anwendungen im Spektrum der CASE-Anwendungen (Coatings, Adhesives, Sealants, Elastomers)
- Halbharte PU-Schaumstoffe

### Beispiele:

### Beispiel 1 (Einstellung des Gehalts an hydrolysierbarem Chlor für monomeres MDI)

Für monomeres MDI wird die Acidität des MDI als Wert für hydrolysierbares Chlor bestimmt.

Es werden 85 Partien eines monomeren MDI nach dem erfindungsgemäßen Verfahren nach den Schritten a) bis c) hergestellt. Die 85 Partien weisen ein Gehalt an 2-Kern-MDI von >98,2 Gew.-% auf. Die einzelnen 2-Kern-MDI-Isomere sind dabei in folgenden Mengen enthalten: 4,4'-MDI (≥ 98,0%), 2,4'-MDI (≤ 2,0%) und 2,2'-MDI (≤ 0,3%) bezogen auf die Summe der 2-Kern-MDI-Isomeren.

In Schritt d) wird der Wert für hydrolysierbares Chlor bestimmt. Die Bestimmung des Werts für hydrolysierbares Chlor erfolgt dabei nach folgender Methode:
Etwa 10 g der MDI-Monomer-Probe wurden in einem 500 ml-Erlenmeyerkolben eingewogen. Dann wurde das MDI-Monomer in siedenden Wasserbad aufgeschmolzen und in 40 ml Chlorbenzol gelöst. Anschließend wurden 100 ml Propanol-(2) zugegeben, der Erlenmeyer-Kolben abgedeckt und das Reaktionsgemisch auf einem Magnetrührer 5 min bei Raumtemperatur gerührt. Dann wurden 100 ml Methanol zugegeben und das Gemisch weitere 5 min bei Raumtemperatur gerührt. Anschließend wurden 100 ml Wasser zugegeben, ein Rückflusskühler aufgesetzt und das Reaktionsgemisch 30 min unter leichtem Rühren zum Sieden erhitzt. Nach Abkühlung wurden 50 ml Aceton durch den Rückflusskühler zugegeben Abschließend wurde der Chlorid-Gehalt des Gemisches durch argentometrische Titration mit einer Silbernitrat-Maßlösung mit einer Stoffmengenkonzentration von ca. 0,002 mol/l unter potentiometrischer Endpunktanzeige titriert.

Bei der Analyse ergibt sich, dass die einzelnen Partien einen Gehalt an hydrolysierbarem Chlor aufweisen, der für die einzelnen Partien stark streut. Der Gehalt an hydrolysierbarem Chlor beträgt für die 85 Partien 27,4 +/- 5,3 mg HCl / kg mit einer relativen Standardabweichung von 19,3 %.

Anschließend werden in Schritt e) die 85 Partien des monomeren MDI durch Zugabe von Isophthalsäuredichlorid auf einen Gehalt an hydrolysierbarem Chlor von 100 mg/kg (gerechnet als HCl, normierte Werte) eingestellt. Dazu wird für jede einzelne Partie die für die Einstellung auf einen Wert für hydrolysierbares Chlor von 100 mg HCl/kg erforderliche Menge an Isophthalsäuredichlorid zudosiert. Dabei erfolgt die Zugabe des Isophthalsäuredichlorids als Masterbatch, gelöst in einem Gemisch aus 45 % 4,4'-MDI und 55 % 2.,4'-MDI. Nach der Einstellung ist die Streuung der Gehalte an hydrolysierbarem Chlor deutlich geringer (100 +/- 3,3 mg HCl / kg ; relative Standardabweichung 3,3 %).

## Patentansprüche

1. Verfahren zur Herstellung von Di- und Polyisocyanaten der Diphenylmethanreihe (MDI) und Gemischen daraus, bei dem man
a) Anilin und Formaldehyd in Gegenwart eines sauren Katalysators zu Di- und Polyaminen der Diphenylmethanreihe umsetzt, und
b) die Di- und Polyamine der Diphenylmethanreihe gegebenenfalls in Gegenwart eines Lösungsmittels phosgeniert, wobei man ein rohes Di- und Polyisocyanat erhält, und
c) aus dem rohen Di- und Polyisocyanat destillativ wenigstens eine Fraktion enthaltend mindestens 5 Gew.-% 3-Kern- und höherkerniges MDI sowie wenigstens eine Fraktion enthaltend mindestens 95 Gew.-% 2-Kern-MDI abtrennt, und
d) den Wert für hydrolysierbares Chlor der Fraktion enthaltend mindestens 95 Gew.-% 2-Kern-MDI analytisch bestimmt, und
e) die Fraktion enthaltend mindestens 95 Gew.-% 2-Kern-MDI mit einer sauren Verbindung, die organische Säurechloride und/oder N-Chloracetamid umfasst, versetzt, so dass der gewünschte Wert für hydrolysierbares Chlor gezielt eingestellt wird.

2. Verfahren nach Anspruch 1, bei dem die saure Verbindung in Schritt e) Essigsäurechlorid, Trichloressigsäurechlorid, Benzoylchlorid, Phthalsäuredichlorid, Terephthalsäuredichlorid und/oder Isophthtalsäuredichlorid umfasst.

3. Verfahren nach einem der Ansprüche 1 oder 2, bei dem die saure Verbindung in Schritt e) aliphatische, aromatische oder cycloaliphatische Carbaminsäurechloride umfasst.

4. Verfahren nach Anspruch 3, bei dem man als Carbaminsäurechlorid N-Phenylcarbamylchlorid oder Butylcarbamylchloride einsetzt.

5. Verfahren nach Anspruch 4, bei dem man als Carbaminsäurechlorid tert.-Butylcarbamylchlorid einsetzt.

6. Verfahren nach einem der Ansprüche 1 bis 3, bei dem die saure Verbindung in Schritt e) p-Toluolsulfonsäurechlorid umfasst.

## Claims

1. Process for preparing di- and polyisocyanates of the diphenylmethane series (MDI) and mixtures thereof, in which
a) aniline and formaldehyde are reacted in the presence of an acidic catalyst to give di- and polyamines of the diphenylmethane series, and
b) the di- and polyamines of the diphenylmethane series are phosgenated, optionally in the presence of a solvent, to obtain a crude di- and polyisocyanate, and
c) at least one fraction containing at least 5% by weight of tricyclic and higher polycyclic MDI and at least one fraction containing at least 95% by weight of bicyclic MDI are separated by distillation from the crude di- and polyisocyanate, and
d) the hydrolysable chlorine value of the fraction containing at least 95% by weight of bicyclic MDI is determined analytically, and
e) the fraction containing at least 95% by weight of bicyclic MDI is admixed with an acidic compound comprising organic acid chlorides and/or N-chloroacetamide, in such a way that the desired hydrolysable chlorine value is established in a controlled manner.

2. Process according to Claim 1, in which the acidic compound in step e) comprises acetyl chloride, trichloroacetyl chloride, benzoyl chloride, phthaloyl chloride, terephthaloyl chloride and/or isophthaloyl chloride.

3. Process according to either of Claims 1 and 2, in which the acidic compound in step c) comprises aliphatic, aromatic or cycloaliphatic carbamoyl chlorides.

4. Process according to Claim 3, in which the carbamoyl chloride used is N-phenylcarbamyl chloride or butylcarbamyl chlorides.

5. Process according to Claim 4, in which the carbamoyl chloride used is tert-butylcarbamyl chloride.

6. Process according to any of Claims 1 to 3, in which the acidic compound in step e) comprises p-toluenesulfonyl chloride.

## Revendications

1. Procédé pour la préparation de diisocyanates et de polyisocyanates de la série du diphénylméthane (MDI) et de mélanges correspondants, dans lequel
a) on transforme de l'aniline et du formaldéhyde en présence d'un catalyseur acide en diamines et en polyamines de la série du diphénylméthane, et
b) on traite avec du phosgène les diamines et les polyamines de la série du diphénylméthane éventuellement en présence d'un solvant, où on obtient un diisocyanate brut et un polyisocyanate brut, et
c) on sépare par distillation du diisocyanate brut et du polyisocyanate brut au moins une fraction contenant au moins 5 % en poids de MDI à 3 noyaux et d'un nombre de noyaux supérieur ainsi qu'au moins une fraction contenant au moins 95 % en poids de MDI à 2 noyaux, et
d) on détermine de manière analytique la valeur pour le chlore hydrolysable de la fraction contenant au moins 95 % en poids de MDI à 2 noyaux, et
e) on mélange la fraction contenant au moins 95 % en poids de MDI à 2 noyaux avec un composé acide, qui comprend des chlorures d'acide organique et/ou un N-chloracétamide, de sorte que la valeur souhaitée pour le chlore hydrolysable soit ajustée de manière ciblée.

2. Procédé selon la revendication 1, dans lequel le composé acide dans l'étape e) comprend du chlorure d'acide acétique, du chlorure d'acide trichloroacétique, du chlorure de benzoyle, du dichlorure d'acide phtalique, du dichlorure d'acide téréphtalique et/ou du dichlorure d'acide isophtalique.

3. Procédé selon l'une quelconque des revendications 1 et 2, dans lequel le composé acide dans l'étape e) comprend des chlorures d'acides carbamiques aliphatiques, aromatiques ou cycloaliphatiques.

4. Procédé selon la revendication 3, dans lequel on utilise du chlorure de N-phénylcarbamyle ou des chlorures de butylcarbamyle en tant que chlorure d'acide carbamique.

5. Procédé selon la revendication 4, dans lequel on utilise du chlorure de tert-butylcarbamyle en tant que chlorure d'acide carbamique.

6. Procédé selon l'une quelconque des revendications 1 à 3, dans lequel le composé acide dans l'étape e) comprend du chlorure d'acide p-toluènesulfonique.
